# EUROPÄISCHE PATENTANMELDUNG

(11) **EP 2 042 098 A1**
(43) Veröffentlichungstag der Anmeldung: **01.04.2009**
(21) Anmeldenummer: 07018880.0
(22) Anmeldetag: 26.09.2007
(51) Int. Cl.: A61B 5/151

(54) **Lanzettenmagazin**

(71) Anmelder: Roche Diagnostics GmbH, 68305 Mannheim (DE); F.HOFFMANN-LA ROCHE AG, 4070 Basel (CH)
(72) Erfinder: Sacherer, Klaus Dieter, 67281 Kirchheim (DE)
(74) Vertreter: Twelmeier, Ulrich

(57) **Zusammenfassung**

Die Erfindung betrifft ein Lanzettenmagazin (1) mit mehreren Lanzetten (7) zum Erzeugen einer Stichwunde zur Gewinnung einer Körperflüssigkeitsprobe.

Erfindungsgemäß ist vorgesehen, dass die Lanzetten (7) in dem Lanzettenmagazin (1) jeweils mit einem Antriebsmechanismus (8) gekoppelt sind, der für jede Lanzette (7) mindestens ein vorgespanntes Antriebselement (8) umfasst, das bei Auslösen des Antriebsmechanismus mechanische Energie zum Beschleunigen der mit ihm gekoppelten Lanzette (7) für eine Stechbewegung freisetzt.

## Beschreibung

Die Erfindung betrifft ein Lanzettenmagazin mit mehreren Lanzetten zum Erzeugen einer Stichwunde zur Gewinnung einer Körperflüssigkeitsprobe. Derartige Lanzettenmagazine werden mit dazu passenden Stechgeräten benutzt, welche die in einem Lanzettenmagazin enthaltenen Lanzetten nacheinander für einen Stich verwenden.

Lanzettenmagazine und dazu passende Stechgeräte werden beispielsweise von Diabetikern verwendet, die mehrmals täglich ihren Blutzuckerspiegel bestimmen müssen und dafür eine aus einer Stichwunde gewonnene Körperflüssigkeitsprobe, in der Regel Blut und/oder interstitielle Flüssigkeit, benötigen.

Ein Stechsystem bestehend aus einem Stechgerät und dazu passenden Lanzettenmagazinen ist beispielsweise aus der DE 10 2004 059 491 A1 bekannt. Bei diesem Stechsystem wird ein Trommelmagazin verwendet, in dem sechs Lanzetten enthalten sind. Das Stechgerät enthält einen Lanzettenantrieb mit einem von einer Antriebsfeder angetriebenen Antriebsrotor, der bei einem Stich einen mit dem Rotor gekoppelten Stößel in Stichrichtung vorschiebt und wieder zurückzieht. Der Stößel wird dabei durch eine Einschuböffnung in das Lanzettenmagazin eingeführt, koppelt dort mit einer Lanzette und schiebt diese aus einer gegenüberliegenden Austrittsöffnung des Lanzettenmagazins heraus, so dass in einem an eine Stechöffnung des Geräts angelegten Körperteil eine Stichwunde erzeugt werden kann. Eine formschlüssige Kopplung zwischen dem Stößel und der Lanzette sorgt dabei dafür, dass die Lanzette bei einer an eine Vorschubbewegung anschließenden Rückführbewegung wieder in das Lanzettenmagazin zurückgezogen wird. Das bekannte Stechsystem bietet einen hohen Benutzerkomfort, jedoch sind die Herstellungskosten sowohl für das Gerät als auch für die dazu passenden Lanzettenmagazine erheblich.

Aufgabe der Erfindung ist es, einen Weg aufzuzeigen, wie ein Stechsystem mit geringerem Aufwand verwirklicht werden kann.

Diese Aufgabe wird durch ein Lanzettenmagazin mit den im Anspruch 1 angegebenen Merkmalen gelöst. Vorteilhafte Weiterbildungen der Erfindung sind Gegenstand der Unteransprüche. Die Aufgabe wird ferner auch durch ein zu einem derartigen Lanzettenmagazin passendes Stechgerät mit den im Anspruch 9 angegebenen Merkmalen sowie ein Stechsystem mit den im Anspruch 10 angegebenen Merkmalen gelöst.

Erfindungsgemäß sind die Lanzetten jeweils mit einem Antriebsmechanismus gekoppelt, der für jede Lanzette ein vorgespanntes Antriebselement umfasst, das bei Auslösen des Antriebsmechanismus mechanische Energie zum Beschleunigen der mit ihm gekoppelten Lanzette für eine Stechbewegung freisetzt.

Obwohl auf diese Weise im Gegensatz zu herkömmlichen Stechsystemen, bei denen nur ein einziger Antriebsmechanismus vorgesehen ist, mit dem die Lanzetten eines Magazins nacheinander koppeln, für jede Lanzette ein eigener Antriebsmechanismus benötigt wird, ermöglicht die Erfindung überraschenderweise Kosteneinsparungen. Indem jeder Lanzette ein Antriebsmechanismus mit einem vorgespannten Antriebselement zugeordnet ist, lassen sich nämlich sowohl die Ankopplung des Antriebs an eine Lanzette als auch der Antrieb selbst wesentlich vereinfachen.

Die Erfindung hat zudem den Vorteil, dass sich eine aus hygienischen Gründen bedenkliche Mehrfachverwendung einer Lanzette zuverlässig dadurch ausschließen lässt, dass das Antriebselement einer Lanzette nach einem Stich entspannt ist und diese deshalb nicht erneut benutzt werden kann. Ein weiterer Vorteil der Erfindung besteht darin, dass ein erfindungsgemäßes Stechsystem sehr klein und kompakt ausgeführt werden kann und trotzdem einen relativ großen Lanzettenvorrat ermöglicht. Dies ist für Diabetiker, die ihren Blutzuckerspiegel mehrmals täglich überprüfen und deshalb ständig ein Stechgerät mit sich führen müssen, ein großer Vorteil.

Bevorzugt sind die Lanzetten in einem erfindungsgemäßen Lanzettenmagazin ringförmig angeordnet. Auf diese Weise lassen sich eine größere Anzahl Lanzetten, beispielsweise 20 bis 50 Lanzetten, besonders kompakt anordnen. Ein erfindungemäßes Lanzettenmagazin kann aber beispielsweise auch als Stapelmagazin ausgebildet sein. Ein weiterer Vorteil einer ringförmigen Anordnung der Lanzetten ist, dass eine einmal eingestellte Stechtiefe für alle Lanzetten des Lanzettenmagazins genutzt werden kann, indem diese durch Drehen des Lanzettenmagazins nacheinander in eine Gebrauchsposition gebracht werden.

Die Integration des Stechantriebs in das Lanzettenmagazin ermöglicht vorteilhaft flache Stechgeräte, die Benutzer leicht mit sich führen kann.

Ein erfindungsgemäßes Lanzettenmagazin kann mit Testelementen zur Untersuchung von Körperflüssigkeitsproben, die durch einen Lanzettenstich gewonnen werden, kombiniert werden. Die Funktionsweise von Testelementen beruht in der Regel auf Nachweisreagenzien, die bei Anwesenheit eines Analyten, beispielsweise Glucose, eine Nachweisreaktion bewirken, deren Stärke mit der Analytkonzentration korreliert. Gebräuchlich sind insbesondere photometrische Testelemente, bei denen die Nachweisereaktion eine Farbänderung bewirkt, und elektrochemische Testelemente, bei denen die Stärke der Nachweisreaktion als Änderung eines elektrischen Stroms gemessen werden kann. Erfindungsgemäße Lanzettenmagazine können mit geringem Aufwand mit Testfeldern, die geeignete Nachweisreagenzien enthalten, ausgestattet werden. Eine Körperflüssigkeitsprobe kann einem solchen Testfeld beispielsweise mittels eines Kapillarkanals einer Lanzette zugeführt werden.

Weitere Einzelheiten und Vorteile der Erfindung werden anhand von Ausführungsbeispielen unter Bezugnahme auf die beigefügten Zeichnungen erläutert. Gleiche und einander entsprechende Teile sind dabei durch übereinstimmende Bezugszahlen gekennzeichnet. Die im Folgenden erläuterten Merkmale können einzeln und in Kombination zum Gegenstand von Ansprüchen gemacht werden. Es zeigen:
- Figur 1:: ein Ausführungsbeispiel eines Lanzettenmagazins;
- Figur 2:: eine Hälfte des geöffneten Magazingehäuses des in Figur 1 gezeigten Ausführungsbeispiels;
- Figur 3:: die in Figur 2 gezeigte Gehäusehälfte mit eingelegten Lanzetten;
- Figur 4:: eine Detailansicht zu Figur 3 mit gebeugtem Lanzettenarm;
- Figur 5:: eine Ansicht gemäß Figur 4 mit gestrecktem Lanzettenarm;
- Figur 6:: ein Lanzettenkranz für das in Figur 1 dargestellte Magazin vor Vereinzelung der Antriebsarme;
- Figur 7:: der in Figur 6 dargestellte Lanzettenkranz nach Vereinzelung der Antriebsarme;
- Figur 8:: ein weiteres Ausführungsbeispiel eines Lanzettenmagazins bei geöffnetem Magazingehäuse;
- Figur 9:: eine Detailansicht zu Figur 8 vor einem Stich;
- Figur 10:: eine Detailansicht gemäß Figur 9 während eines Stichs,
- Figur 11:: eine Detailansicht gemäß Figur 9 nach einem Stich;
- Figur 12:: eine weitere Detailansicht zu Figur 8; und
- Figur 13: eine Ausführungsbeispiel eines Stechgeräts.

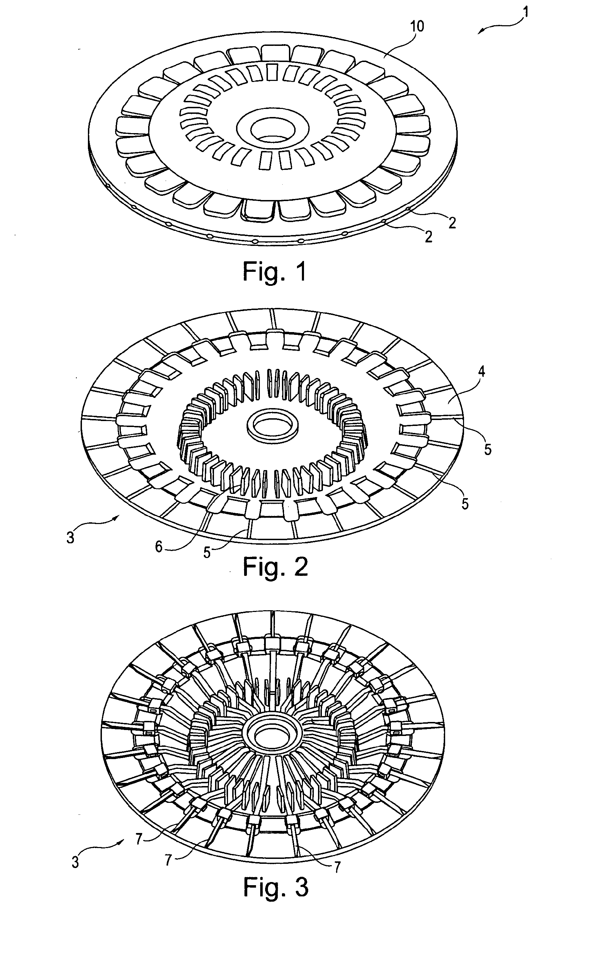

Figur 1 zeigt ein Ausführungsbeispiel eines Lanzettenmagazins 1. Ein derartiges Lanzettenmagazin wird bestimmungsgemäß in ein Stechgerät eingesetzt, mit dem die in dem Lanzettenmagazin 1 enthaltenen Lanzetten nacheinander zum Erzeugen einer Stichwunde verwendet werden. Das scheibenförmige Lanzettenmagazin 1 hat in seiner Umfangsfläche Austrittsöffnungen 2, aus denen in dem Lanzettenmagazin 1 enthaltene Lanzetten bei Gebrauch austreten.

Figur 2 zeigt eine Gehäusehälfte 3 des Lanzettenmagazins 1. Das Gehäuse des Lanzettenmagazins 1 ist aus zwei Gehäusehälften zusammengefügt, die bevorzugt identisch ausgebildet sind, um die Fertigung zu vereinfachen. Die Gehäusehälften können aber auch unterschiedlich sein. Die Gehäusehälften 3 sind, wie in Figur 1 dargestellt, bevorzugt tellerförmig ausgebildet.

Bei zusammengefügtem Gehäuse liegen die Gehäusehälften 3 mit Randflächen 4 aufeinander, die Führungskanäle 5 für die Lanzetten enthalten. Auf diese Weise werden in dem Lanzettenmagazin 1 Kammern geschaffen, in denen die Lanzetten sowohl vor als auch nach Gebrauch einzeln gelagert werden können.

Die bevorzugt als Spritzgussteil aus Kunststoff hergestellten Gehäusehälften 3 können formschlüssig, beispielsweise durch Kleben oder Schweißen, miteinander verbunden werden. Bevorzugt werden die Gehäusehälften 3 jedoch mechanisch mittels Verbindungselementen 6 zusammengefügt.

Bei dem dargestellten Ausführungsbeispiel sind die Verbindungselemente 6 als ein Kranz von Klemmrippen ausgebildet. Beim Zusammenfügen der beiden Gehäusehälften 3 greifen die Klemmrippen 6 der beiden Gehäusehälften 3 ineinander, so dass die beiden Gehäusehälften 3 klemmschlüssig, also durch Reibungskräfte, zusammengehalten werden. Es ist aber auch möglich, die Verbindungselemente 6 für ein formschlüssiges Verbinden der Gehäusehälften 3 auszubilden.

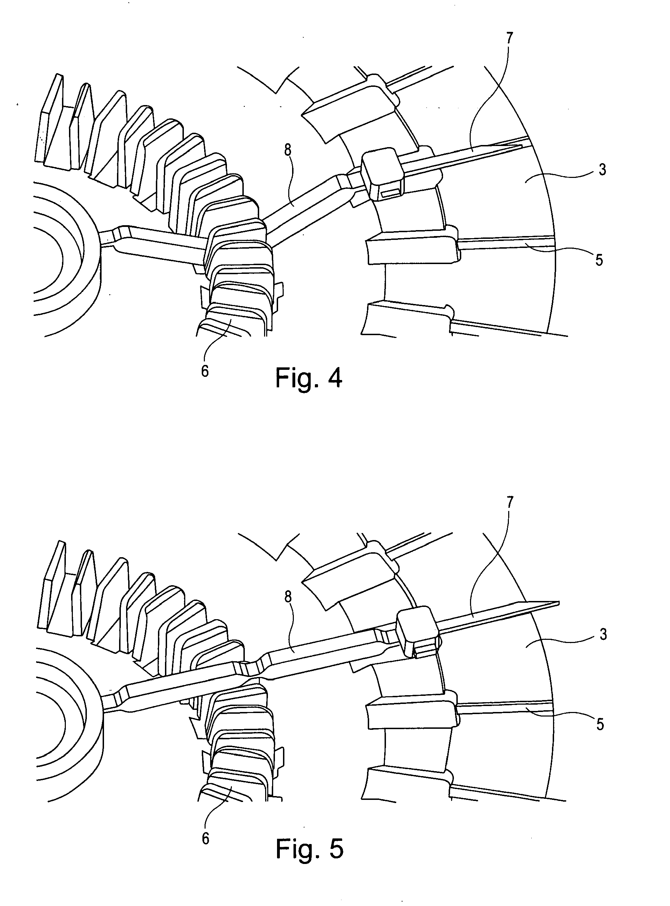

Figur 3 zeigt eine Gehäusehälfte 3 gemäß Figur 2 mit eingesetzten Lanzetten 7. Die Lanzetten 7 liegen in den Führungskanälen 5 der Gehäusehälfte 3 und erstrecken sich in radialer Richtung. Weitere Einzelheiten lassen sich am besten in Figur 4 erkennen, die eine Detailansicht zu Figur 3 zeigt. Zur Vereinfachung ist dabei in Figur 4 nur eine einzige Lanzette 7 dargestellt.

Die Lanzetten 7 sind in dem Lanzettenmagazin 1 jeweils mit einem Antriebsmechanismus gekoppelt. Dieser Antriebsmechanismus umfasst einen Antriebsarm 8, der die Lanzetten 7 trägt. In Figur 4 ist der Antriebsarm 8 in einer gebeugten Ausgangsposition dargestellt. Während der Vorschubphase einer Stechbewegung geht der Antriebsarm 8, der bei dem dargestellten Ausführungsbeispiel ein Kniehebel ist, aus seiner Ausgangsposition in eine gestreckte Position über, die in Figur 5 dargestellt ist. Durch das Strecken des Antriebsarms 8 wird die von ihm getragene Lanzette 7 radial auswärts bewegt, so dass ihre Spitze aus der Austrittsöffnung 2 des Lanzettenmagazins 1 austritt.

Die beschriebene Bewegung des Antriebsarms 8 bewirkt, dass die von ihm getragene Lanzette 7 eine Stichbewegung mit sehr hoher Präzision ausführt. Insbesondere ist der Umkehrpunkt der Lanzettenbewegung, in dem die Vorschubbewegung des Stichs in eine Rückführbewegung übergeht, durch den Antriebsarm 8 sehr genau vorgegeben, so die Stechtiefe sehr präzise eingestellt werden kann.

Der Antriebsarm 8 ist mit einem vorgespannten Antriebselement (nicht dargestellt) gekoppelt, das beispielsweise eine Wendelfeder sein kann, die komprimiert zwischen der Beugung des Lanzettenarms 8 und der in Figur dargestellten Gehäusehälfte 3 angeordnet ist. Wird der Antriebsmechanismus ausgelöst, entspannt sich das Antriebselement und setzt dabei mechanische Energie zum Beschleunigen der mit ihm gekoppelten Lanzette 7 für eine Stechbewegung frei. Anstelle einer Wendelfeder kann als Antriebselement beispielsweise auch ein gespanntes Gummiband verwendet werden, das beispielsweise an der Biegung des Antriebsarms 8 ansetzt und an seinem anderen Ende mit einer aufgesetzten Gehäusehälfte verbunden ist.

Der Antriebsarm 8 wird mit einem geeigneten Sperrmechanismus bis zum Auslösen eines Stichs in der in Figur 4 dargestellten Ausgangsposition gehalten. Der Sperrmechanismus kann beispielsweise durch die Klemmrippen 6 ausgebildet sein, die zwischen sich eine Engstelle ausbilden und auf diese Weise klemmend den Antriebsarm in der in Figur 4 gezeigten Ausgangslage halten. Die von dem vorgespannten Antriebselement ausgeübte Kraft ist dabei so bemessen, dass sie nicht ausreicht, den Antriebsarm 8 durch die Engstelle zu drücken. Zum Auslösen einer Stichbewegung kann ein Auslösestift eines Stechgeräts durch die in Figur 1 dargestellte Auslöseöffnungen 10 hindurch greifen und das vorgespannte Antriebselemente kurzzeitig unterstützen. Die von einem solchen Auslösestift ausgeübte Kraft reicht in Kombination mit der Kraft des vorgespannten Antriebselements aus, den Antriebsarm 8 an der Engstelle vorbeizudrücken und dabei die Klemmrippen 6 seitlich wegzudrücken. Sobald der Antriebsarm 8 die Engstelle zwischen den Klemmelementen 6 passiert hat, wird er von dem Antriebselement weiter beschleunigt, so dass eine Stechbewegung ausgeführt wird.

Das vorgespannte Antriebselement ist bevorzugt so ausgebildet, dass es nach einem Auslösen eines Stichs den Kniehebel 8 aus der in Figur 4 dargestellten Ausgangsposition in die in Figur 5 dargestellte Position und anschließend in eine Endposition bewegt, in welcher der Antriebsarm 8 umgekehrt wie in der Ausgangsposition gebogen ist. Auf diese Weise wird bewirkt, dass die Lanzette 7 nach einem Stich wieder in das Lanzettenmagazin 1 zurückgezogen wird. Eine Stichbewegung der Lanzette 7 gliedert sich also in eine Vorschubphase, in welcher die Lanzette 7 aus dem Lanzettenmagazin 1 heraus geschoben wird, und eine daran anschließende Rückführphase, in der die Lanzette 7 wieder in das Lanzettenmagazin 1 zurückgezogen wird. Der Antriebsarm 8 geht in einer an die Vorschubphase anschließenden Rückführphase aus der in Figur 5 gezeigten gestreckten Position in eine gebeugte Endposition über, deren Beugungsrichtung genau umgekehrt wie in der Ausgangsposition ist.

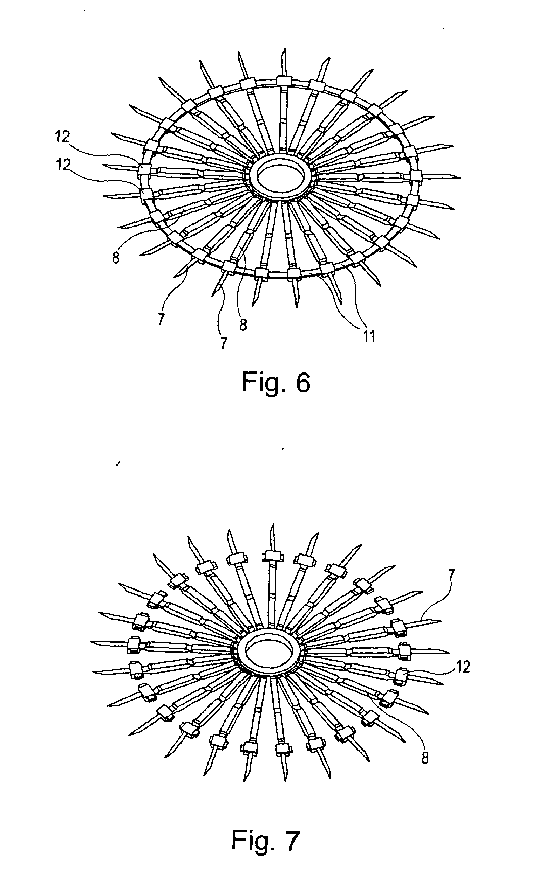

Die Lanzetten 7 und die mit ihnen verbundenen Antriebsarme 8 können prinzipiell einzeln in das Lanzettenmagazin 1 eingesetzt werden. Eine wesentlich rationellere Fertigung lässt sich jedoch erreichen, indem die Lanzetten 7 und die Antriebsarme 8 als Kranz gefertigt und in einem Arbeitsgang in eine Gehäusehälfte 3 des Lanzettenmagazins 1 eingesetzt werden. Figur 6 zeigt ein Ausführungsbeispiel eines Lanzettenkranzes mit Lanzetten 7 und Antriebsarmen 8, der durch Spritzgießen kostengünstig gefertigt werden kann. Die Lanzetten 7 können dabei einstückig mit den übrigen Elementen des Lanzettenkranzes aus einem Hartkunststoff, beispielsweise Polycarbonat, hergestellt werden. Möglich ist es auch, die Lanzetten 7 aus einem anderen Werkstoff, insbesondere aus Edelstahl, herzustellen und in Lanzettenhalterungen 12 des Lanzettenkranzes einzusetzen.

Für die Herstellung und das Einsetzen der Lanzetten 7 in die Lanzettenhalterungen 12 ist es günstig, die Lanzettenhalterungen 12 zunächst als Teile eines umlaufenden Rings auszubilden. Dieser die Lanzettenhalterungen 12 verbindende Ring ist in Figur 6 zu erkennen. Die Lanzettenhalterungen 12 sind dabei über Stege 11 miteinander verbunden.

Vor dem Einsetzen des Lanzettenkranzes in eine Gehäusehälfte 3 werden die Stege 11 zwischen den Lanzettenhalterungen 12 entfernt. Dies kann kostengünstig beispielsweise durch einen Stanzvorgang erfolgen. Figur 7 zeigt einen Lanzettenkranz, bei dem die Stege 11 entfernt wurden und der zum Einsetzen in eine Gehäusehälfte 3 bereit ist.

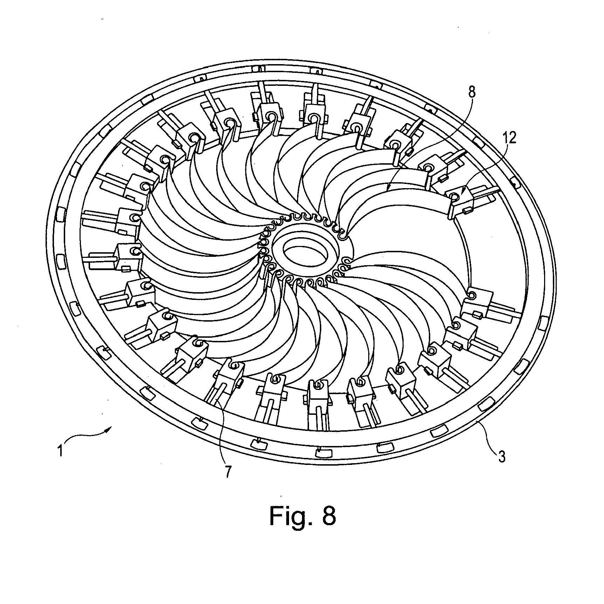

Figur 8 zeigt ein weiteres Ausführungsbeispiel eines Lanzettenmagazins 1 bei geöffnetem Gehäuse. Das in Figur 8 dargestellte Ausführungsbeispiel unterscheidet sich von dem vorstehend beschriebenen Ausführungsbeispiel insbesondere dadurch, dass die Antriebsarme 8 als Blattfedern ausgebildet sind. Während also bei dem unter Bezugnahme auf die Figuren 1 bis 7 beschriebenen Ausführungsbeispiel der Antriebsarm 8 und das vorgespannte Antriebselement, das mit dem gekoppelt ist, separate Teile des Antriebsmechanismus sind, ist bei dem in Figur 8 dargestellten Ausführungsbeispiel der Antriebsarm 8 zugleich auch das vorgespannte Antriebselement.

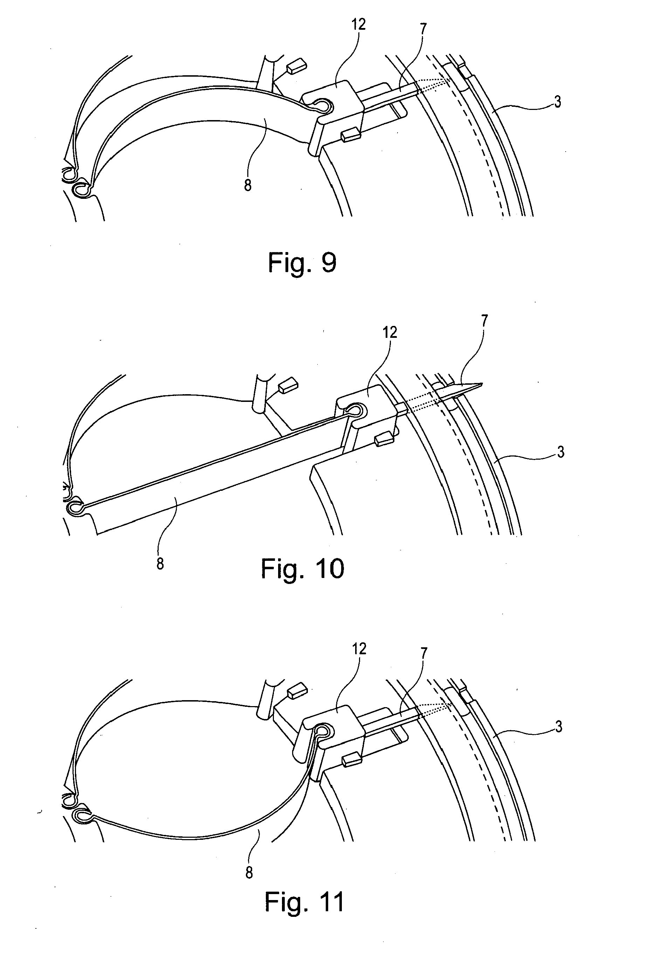

Figur 9 zeigt eine Detailansicht zu Figur 8, in welcher der Antriebsarm 8 in seiner gebeugten Ausgangsposition dargestellt ist. Bei einem Stich geht der Antriebsarm 8 während einer Vorschubphase der Stechbewegung aus dieser gebeugten Ausgangsposition in eine gestreckte Position über, die in Figur 10 gezeigt ist. Die den Antriebsarm 8 bildende Blattfeder ist dabei so bemessen, dass die gestreckte Position keine entspannte Gleichgewichtsposition ist. Der Antriebsarm entspannt sich deshalb über die gestreckte Position hinaus und geht in eine gebeugte Endposition über, die in Figur 11 gezeigt ist. Erst in dieser Endposition ist der Antriebsarm 8 entspannt. In der in Figur 11 gezeigten Endposition ist der Antriebsarm 8 in umgekehrter Richtung wie in der in Figur 9 gezeigten Ausgangsposition gebeugt.

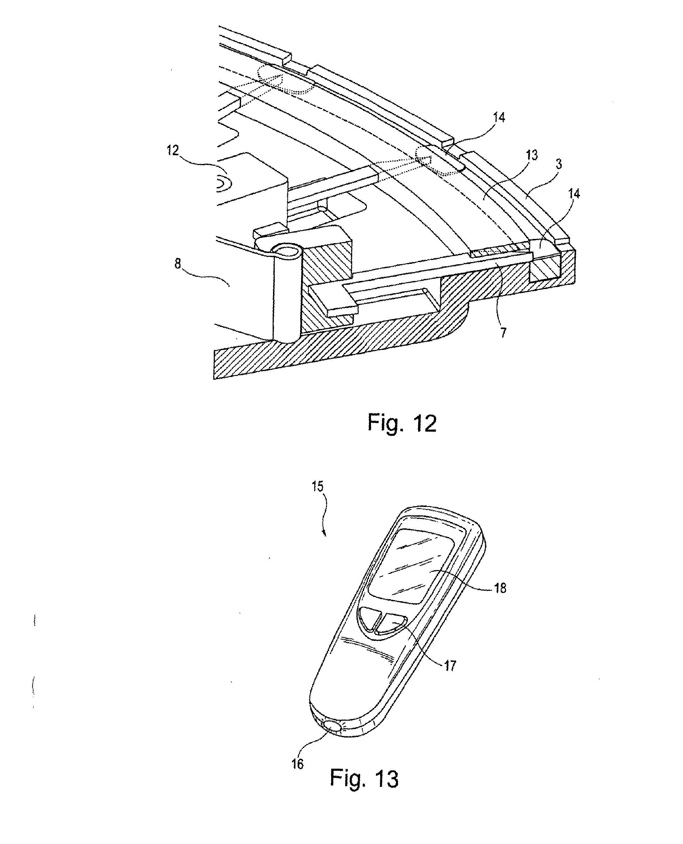

Die Spitzen der Lanzetten 7 sind durch einen ringförmigen Sterilschutz 13 vor Kontamination geschützt. Der Sterilschutz 13 kann beispielsweise aus Silikon sein, das nach dem Einlegen der Lanzetten 7 in die Gehäusehälfte 3 flüssig auf die Lanzetten 7 aufgebracht wird und sich anschließend verfestigt. Der Sterilschutz 13 kann auch als eine ring- oder scheibenförmige Folie ausgebildet sein, die zum Schutz der Lanzetten 7 auf die Lanzettenspitzen aufgebracht wird. Figur 12 zeigt eine Detailansicht zu Figur 8, in welcher der Sterilschutz 13 zu sehen ist.

Die vorstehend beschriebenen Lanzettenmagazine 1 können zusätzlich mit Testfeldern zur Untersuchung von Körperflüssigkeitsproben, die durch einen Lanzettenstich gewonnen wurden, ausgerüstet sein. Derartige Testfelder 14 sind in Figur 12 zu erkennen. Die Testfelder 14 weisen bevorzugt Nachweisreagenzien auf, die eine photometrische oder elektrochemische Bestimmung einer Analytkonzentration, beispielsweise der Glucosekonzentration, ermöglichen. Entsprechende Testfelder sind bei handelsüblichen Teststreifen, beispielsweise zur Blutzuckerbestimmung, vorhanden und bedürfen deshalb keiner weiteren Erläuterung.

Die Testfelder 14 sind durch Trockenmittel 19 vor einer Beeinträchtigung durch Feuchtigkeit geschützt. Das Trockenmittel 19 kann beispielsweise in einer Ringnut einer Gehäusehälfte 3 angeordnet und von Testfeldern 14 bedeckt sein. Bevorzugt ist das Trockenmittel in einzelnen Vertiefungen angeordnet, die jeweils von einem Testfeld 14 bedeckt sind. Auf diese Weise können die Testfelder 14 und die Lanzetten 7 in einzelnen Kammern des Lanzettenmagazins gelagert werden. Eine Beeinträchtigung unbenutzter Lanzetten 7 und unbenutzter Testfelder 14 durch Keime und/oder Körperflüssigkeit benutzter Lanzetten 7 oder Testfelder 14 kann so ausgeschlossen werden.

Zur photometrischen Auswertung einer mit einem Testfeld 14 und einer Körperflüssigkeitsprobe durchgeführten Nachweisreaktion sind in der die Testfelder 14 bedeckenden Gehäusehälfte Öffnungen angeordnet, die einen Durchtritt von Messlicht zwischen einem Testfeld 14 und einer Messeinrichtung eines Stechgeräts ermöglichen.

Testfelder 14, Trockenmittel 19 und eine die Lanzetten 7 schützende Sterilschutzschicht, vorzugsweise eine Folie, können ring- oder scheibenförmig kompakt und kostengünstig an dem vorstehend beschriebenen Lanzettenmagazinen 1 angebracht werden.

Figur 13 zeigt in einer schematischen Darstellung ein Ausführungsbeispiel eines Stechgeräts 15, in das die vorstehend beschriebenen Lanzettenmagazine 1 einsetzbar sind. Das Stechgerät 15 hat ein Aufnahmefach (nicht dargestellt) für ein Lanzettenmagazin 1. Das Aufnahmefach hat eine verschließbare Öffnung, die sich auf der Rückseite des in Figur 13 dargestellten Ausführungsbeispiels befindet.

Das Stechgerät 15 weist eine Geräteöffnung 16 auf, gegen die ein Körperteil zum Erzeugen einer Stichwunde gepresst wird. Das Stechgerät hat ferner Bedienungselemente 17 in Form von Tasten und eine Anzeigeeinrichtung 18 in Form einer Flüssigkristallanzeige zum Anzeigen von Untersuchungsergebnissen.

Ein in das Stechgerät 15 eingesetzte Lanzettenmagazin 1 wird mittels einer Transporteinrichtung schrittweise gedreht, so dass die in dem Lanzettenmagazin enthaltenen Lanzetten nacheinander für einen Stich in ein an die Geräteöffnung 16 angelegtes Körperteil genutzt werden können. Die Transporteinrichtung kann manuell oder von einem Elektromotor angetrieben werden.

Das in Figur 13 dargestellte Stechgerät 15 bildet zusammen mit einem Lanzettenmagazin 1 ein Stechsystem. Ein erfindungsgemäßes Stechsystem kann jedoch auch als ein Wegwerfgerät ausgebildet sein, bei dem ein Austausch der Lanzetten nicht möglich ist und das deshalb entsorgt wird, sobald alle in ihm enthaltenen Lanzetten benutzt wurden. Ein derartiges Wegwerfstechsystem hat mehrere in einem Gehäuse angeordnete Lanzetten, die jeweils mit einem Antriebsmechanismus gekoppelt sind, der für jede Lanzette mindestens ein vorgespanntes Antriebselement umfasst, das bei Auslösen des Antriebsmechanismus mechanische Energie zum Beschleunigen der mit ihm gekoppelten Lanzette für eine Stechbewegung freisetzt.

### Bezugszahlen

- 1: Lanzettenmagazin
- 2: Austrittsöffnungen
- 3: Gehäusehälfte
- 4: Randflächen
- 5: Führungskanäle
- 6: Verbindungselemente / Klemmrippen
- 7: Lanzetten
- 8: Antriebsarm
- 10: Auslöseöffnungen
- 11: Stege
- 12: Lanzettenhalterungen
- 13: Sterilschutz
- 14: Testfeld
- 15: Stechgerät
- 16: Geräteöffnung
- 17: Bedienungselemente
- 18: Anzeigeeinrichtung
- 19: Trockenmittel

## Patentansprüche

1. Lanzettenmagazin mit mehreren Lanzetten (7) zum Erzeugen einer Stichwunde zur Gewinnung einer Körperflüssigkeitsprobe, **dadurch gekennzeichnet, dass** die Lanzetten (7) in dem Lanzettenmagazin (1) jeweils mit einem Antriebsmechanismus (8) gekoppelt sind, der für jede Lanzette (7) mindestens ein vorgespanntes Antriebselement (8) umfasst, das bei Auslösen des Antriebsmechanismus mechanische Energie zum Beschleunigen der mit ihm gekoppelten Lanzette (7) für eine Stechbewegung freisetzt.

2. Lanzettenmagazin nach Anspruch 1, **dadurch gekennzeichnet, dass** der Antriebmechanismus einen Antriebsarm (8) umfasst, der jeweils eine der Lanzetten (7) trägt und während einer Vorschubphase einer Stechbewegung aus einer gebeugten Ausgangsposition in eine gestreckte Position übergeht.

3. Lanzettenmagazin nach Anspruch 2, **dadurch gekennzeichnet, dass** der Antriebsarm (8) in einer an die Vorschubphase anschließenden Rückführphase, in der eine Lanzette (7) zurückgezogen wird, aus der gestreckten Position in eine gebeugte Endposition übergeht.

4. Lanzettenmagazin nach Anspruch 2 oder 3, **dadurch gekennzeichnet, dass** der Antriebsarm (8) ein Kniehebel ist.

5. Lanzettenmagazin nach einem der Ansprüche 2 bis 4, **dadurch gekennzeichnet, dass** der Antriebsarm (8) und das vorgespannte Antriebselement separate Teile des Antriebsmechanismus sind.

6. Lanzettenmagazin nach einem der Ansprüche 2 bis 4, **dadurch gekennzeichnet, dass** der Antriebsarm (8) das vorgespannte Antriebselement ist.

7. Lanzettenmagazin nach einem der vorstehenden Ansprüche, **dadurch gekennzeichnet, dass** die Lanzetten (7) in dem Lanzettenmagazin (1) ringförmig angeordnet sind.

8. Lanzettenmagazin nach einem der vorstehenden Ansprüche, **gekennzeichnet durch** ein Magazingehäuse (3), das für jede Lanzette (7) jeweils eine Austrittsöffnung (2) aufweist, **durch** welche bei einer Stechbewegung die Spitze einer der Austrittsöffnung (2) zugeordneten Lanzette (7) hindurchtritt.

9. Stechgerät mit
einem Aufnahmefach für ein Lanzettenmagazin (1) nach einem der vorstehenden Ansprüche,
einer Gehäuseöffnung (16) zum Anlegen an ein Körperteil, in dem eine Stichwunde erzeugt werden soll, und
einem Transportmechanismus, um die in einem in das Aufnahmefach eingelegten Lanzettenmagazin (1) enthaltenen Lanzetten (7) nacheinander in eine Gebrauchsposition zu bewegen, in der sie zum Erzeugen einer Stichwunde in einem an die Gehäuseöffnung (16) angelegten Körperteil eine Stechbewegung beginnen.

10. Stechsystem mit mehreren in einem Gehäuse (3) angeordneten Lanzetten (7), die jeweils mit einem Antriebsmechanismus (8) gekoppelt sind, der für jede Lanzette (7) mindestens ein vorgespanntes Antriebselement umfasst, das bei Auslösen des Antriebsmechanismus mechanische Energie zum Beschleunigen der mit ihm gekoppelten Lanzette (7) für eine Stechbewegung freisetzt.
